# EUROPEAN PATENT APPLICATION

(11) **EP 3 620 197 A1**
(43) Date of publication of application: **11.03.2020**
(21) Application number: 19188785.0
(22) Date of filing: 29.07.2019
(51) Int. Cl.: A61M 25/00

(54) **TUBULAR BODY**

(30) Priority: 05.09.2018 JP 2018165635
(71) Applicant: Asahi Intecc Co., Ltd., Seto-shi, Aichi 489-0071 (JP)
(72) Inventor: NISHIHARA, Hiroyuki, Seto-shi, Aichi, 489-0071 (JP); TAKECHI, Aya, Seto-shi, Aichi, 489-0071 (JP)
(74) Representative: Winter, Brandl, Fürniss, Hübner, Röss, Kaiser, Polte - Partnerschaft mbB

(57) **Abstract**

[PROBLEM TO BE SOLVED]

When one end portion of a tubular body is operated, the tubular body shall prevent a deviation of wires, improve a torque transmissibility and pushing force to the other end portion of the tubular body, and improve insertability of a medical device into an interior of the tubular body. Further, the tubular body shall improve flexibility and durability.

[SOLUTION]

A tubular body 1 is configured from eight first wires (3a ∼ 3h) and eight second wires (5a ∼ 5h) twisted into a hollow tubular configuration so that the first wires and second wires are alternately arranged with each other, wherein each of the first wires (3a ∼ 3h) has a substantially circular cross-section, each of the second wires (5a ∼ 5h) has recesses in contact portions opposite to adjacent first wires (5ar, 5as, 5br, 5bs, 5cr · · · 5hs), and has a convex outer arc (5ap, 5bp etc.) and a convex inner arc (5aq, 5bq etc.) in a cross-sectional view.

## Description

### [Technical Field]

The present invention relates to a tubular body that is configured by twisting a plurality of wires.

### [Background Art]

Conventionally, in a catheter percutaneously inserted into a blood vessel, from the viewpoint of the performance of pushability for transmitting pushing force of an operator to a distal end portion of the catheter, torque transmissibility for transmitting a rotative force (torque) to the distal end portion of the catheter when the operator rotates a proximal end portion of the catheter, kink resistance not causing bend at the bent portion or the curved portion of the blood vessel, and insertability of another medical devices into the catheter lumen and so on, various medical tube suitable for catheters have been developed.

For example, Patent Document 1 discloses a medical tube including a coil layer in which a flat wire (its cross section is rectangular) is densely wound is arranged on an inner layer of the medical tube, and an outer layer of polyamide elastomer is arranged on an outside of the coil layer (see FIG. 1 etc.). Patent Document 1 also discloses that it is possible to achieve all of flexibility, good kink resistance, and good tensile strength according to this medical tube (see paragraph [0006] etc.).

### [Prior art documents]

### [Patent Document]

[Patent Document 1] JP 2010-136895 A

### [Problems that the Invention is to solve]

### [Summary of the Invention]

However, with the coil used in the medical tube disclosed in Patent Document 1, since the wire wound is flat wire (its cross section is rectangular), when one end portion of the medical tube is operated, there was a problem that a portion of the wire deviates in cross section radially inwards or outwards of the coil from adjacent wire portions.

Therefore, in the medical tube disclosed in Patent Document 1, in order to prevent the deviation of the wire windings, the outer tube made of polyamide elastomer is coated on the outside of the coil (see paragraph [0042] etc.).

Incidentally, when the user manipulates one end portion of the medical tube, a problem that a portion of the wire deviates in cross section radially inwards or outwards of the coil against the wire portions adjacent to both sides, has occurred even if the wire forming the coil is a round wire (circular cross section), i.e., not only in the case of a flat wire (rectangular cross section).

Incidentally, when the wire constituting the coil is a round wire (circular cross section) compared with the case when the wire constituting the coil is a flat wire, because the inner surface of the coil is more uneven, a problem that the insertion resistance of a medical device inserted into an inside of the coil is even worse has occurred.

The present invention has been made in response to the above problems the aforementioned prior art has. When the user manipulates one end portion of the coil (hereinafter, referred to as "tubular body"), the present invention may prevent a misalignment between the wires, may improve the torque transmissibility and pushing force to the other end portion of the tubular body, and may improve insertability of the medical device into the inside of the tubular body, and further may improve flexibility and improve durability.

### [Means for Solving the Problems]

The problems described above are solved by a tubular body according to claim 1. Claims 2 to 7 relate to preferred embodiments of a tubular body in accordance with the present invention. Further, claim 8 relates to a method for manufacturing a tubular in accordance with the present invention.

A tubular body in accordance with the present invention comprises a plurality of first wires and a plurality of second wires, wherein the first wires and the second wires are twisted into a tubular configuration so that the first wires and second wires are alternately arranged with each other and each of the second wires is placed between two adjacent first wires, and wherein at least one of the first wires has a substantially circular, i.e. round, cross section perpendicular to a longitudinal direction of the tubular body, and at least one of the second wires has a cross section perpendicular to the longitudinal direction with a recess in a side surface opposite to the first wire, and the cross section of the second wire is annular sector shaped and projects radially outwardly.

Preferably, each of the first wires may have a round cross section perpendicular to the longitudinal direction, each of the second wires may have a cross section perpendicular to the longitudinal direction with the recess in one side surface opposite to the first wire and another recess in the other side surface opposite to the other first wire, and the cross section of each of the second wires may be annular sector shaped and project radially outwardly.

In a preferred embodiment, a radially outer end of the recess of the second wire may contact with a radially outer end of the recess of the adjacent second wire, and a radially inner end of the recess of the second wire may contact with a radially inner end of the recess of the adjacent second wire.

In another preferred embodiment, a radially outer end of the recess of the second wire may be spaced apart from a radially outer end of the recess of the adjacent second wire, and a radially inner end of the recess of the second wire is spaced apart from a radially inner end of the recess of the adjacent second wire.

Further, each of the plurality of second wires may be a multi-strand wire configured from a plurality of third wires twisted with each other.

Further, each of the plurality of first wires may be a multi-strand wire configured from a plurality of fourth wires twisted with each other.

Furthermore, each of the plurality of the second wires may be formed by deforming a wire of larger outer diameter than an outer diameter of the first wire.

Furthermore, each of the second wires may be made of a material that is softer than a material of the first wires.

A method for manufacturing a tubular body in accordance with the present invention may comprise the following steps:
twisting a plurality of first wires having a round cross section perpendicular to a longitudinal direction of the tubular body, and a plurality of second wires having a round cross section perpendicular to the longitudinal direction around a core wire so that the first wires and second wires are alternately arranged with each other and each of the second wires is placed between two of the first wires, wherein a diameter of the round cross section of the second wire is greater than a diameter of the round cross section of the first wires;
pressing the second wires from radially outside so that the round cross section of each of the second wires is deformed into an annular sector shape projecting radially outwardly and having a recess in a side surface opposite to the first wire; and
pulling the core wire from the tubular body.

### [Effects of the Invention]

With a tubular body in accordance with the present invention, when the user manipulates one end portion of the tubular body, it is possible to prevent deviation between the first wires and second wires, to improve torque transmissibility and pushing force to the other end portion of the tubular body. Further, it is possible to improve insertability of a medical device into the inside of the tubular body.

Further, where each of the plurality of second wires is configured from a plurality of third wires twisted with each other, when the tubular body is curved, by subtle movement of the third wires, flexibility and durability may be improved.

Further, where each of the plurality of first wires is configured from a plurality of fourth wires twisted with each other, when the tubular body is curved, by subtle movement of the fourth wires, flexibility and durability may also be further improved.

Further, where each of the plurality of the second wires is formed by deforming a wire of larger outer diameter than an outer diameter of the first wire, the second wire is sufficiently filled between the first wires, it is possible to prevent a deviation between the first wires and second wires and to improve torque transmission and pushing force to the other end portion of the tubular body when the user manipulates one end portion of the tubular body.

### [BRIEF DESCRIPTION OF THE DRAWINGS]

FIG. 1 is a perspective view of the tubular body of the first embodiment of the present invention.
FIG. 2 is a cross-sectional view of the tubular body of the first embodiment.
FIG. 3 is a partially enlarged view of FIG. 2.
FIG. 4 is a perspective view of the tubular body of the second embodiment.
FIG. 5 is a cross-sectional view of the tubular body of the second embodiment.
FIG. 6 is a partially enlarged view of FIG. 5.
FIG. 7 is a cross-sectional view showing an initial state of setting a stranded wire to a swaging machine in producing a tubular body of the second embodiment.
FIG. 8 is a cross-sectional view showing an intermediate state in producing the tubular body of the second embodiment.
FIG. 9 is a cross-sectional view showing a final state in producing the tubular body of the second embodiment.
FIG. 10 is a cross-sectional view of the tubular body of the third embodiment.
FIG. 11 is a partially enlarged view of FIG. 10.
FIG. 12 is a cross-sectional view of the tubular body of the fourth embodiment.
FIG. 13 is a partially enlarged view of FIG. 12.
FIG. 14 is a cross-sectional view of the tubular body of the fifth embodiment.
FIG. 15 is a partially enlarged view of FIG 14.

### [EMBODIMENTS OF THE PRESENT INVENTION]

Hereinafter, embodiments of the present invention will be described with reference to the drawings.

### (First Embodiment)

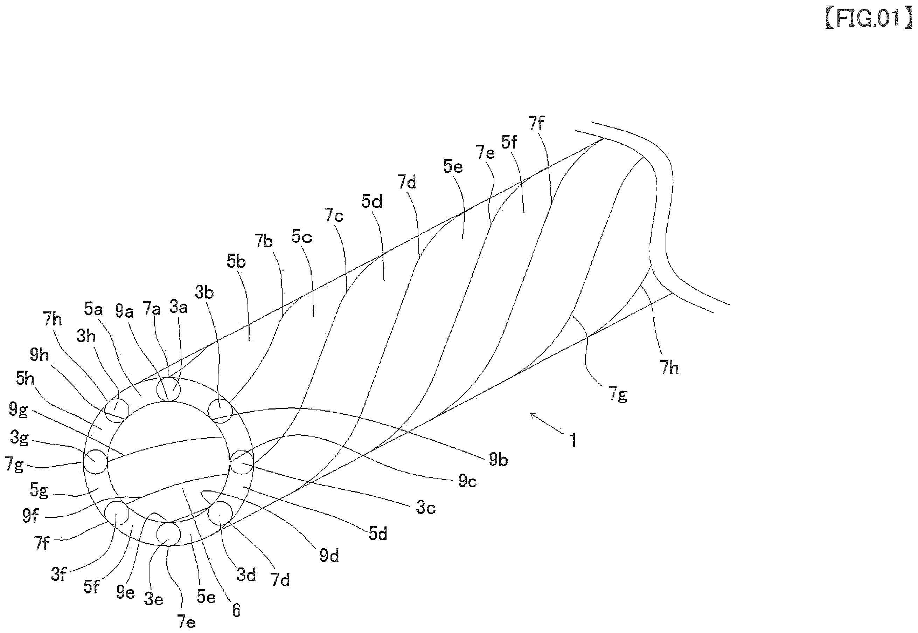

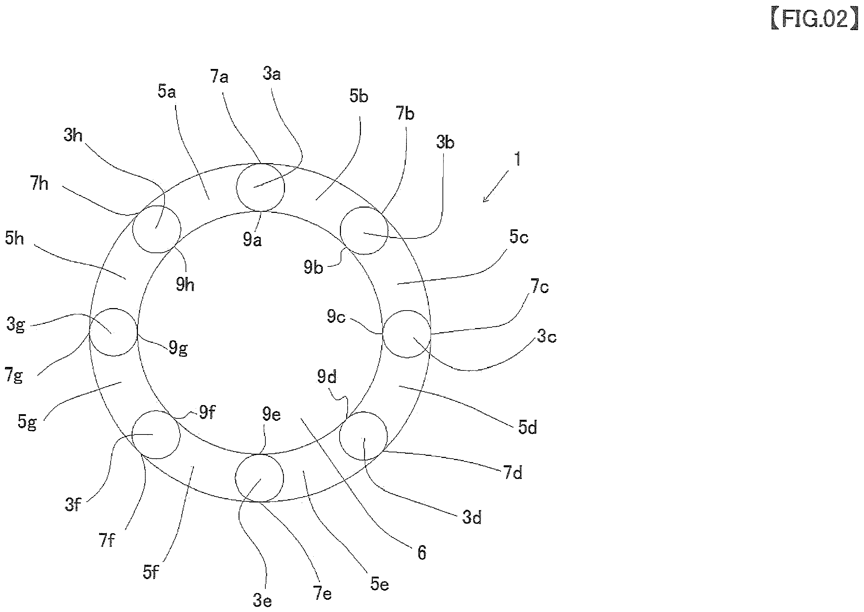

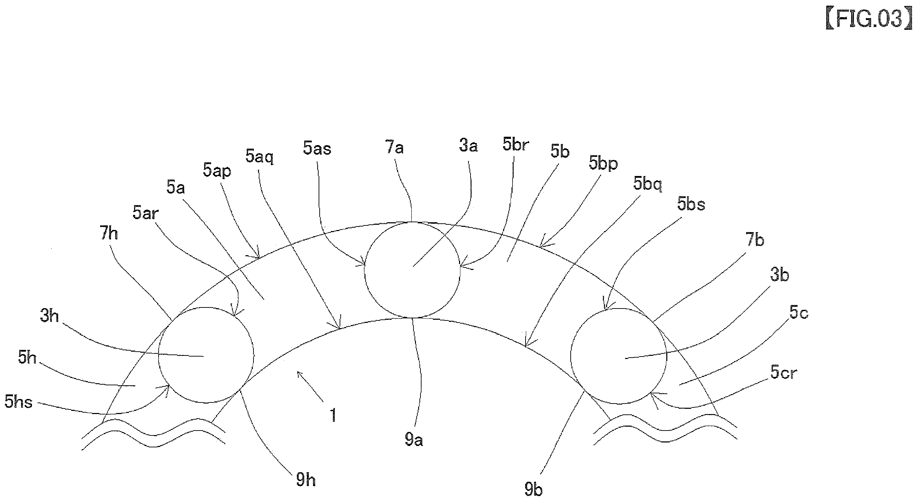

Firstly, a first embodiment of the present invention will be described. Figure 1 is a perspective view of the tubular body of the first embodiment of the present invention, FIG. 2 is a cross-sectional view of the tubular body of the first embodiment, FIG. 3 is a partially enlarged view of FIG. 2.

As shown in FIG. 1, a tubular body 1 is an elongated tubular body configured from eight first wires (3a, 3b, 3c, 3d, 3e, 3f, 3g, 3h) and eight second wires (5a, 5b, 5c, 5d, 5e, 5f, 5g, 5h) which are spirally twisted into a hollow tubular configuration so that the first wires and second wires are alternately arranged with each other and each of the first wires is placed between two adjacent second wires.

Further, the tubular body 1 includes a substantially circular outer peripheral surface and a substantially circular inner peripheral surface in a cross-sectional view, and the tubular body 1 forms a hollow portion 6 in an inside of the inner peripheral surface thereof.

A first wire of the present embodiment (3a ∼ 3h) is substantially circular, i.e. round, in a cross-sectional view. Here, the reason why the first wires (3a ∼ 3h) are described as substantially circular and not exactly circular in a cross-sectional view depends on considering a case where the first wire is slightly deformed by the manufacturing method of the tubular body described below. However, the first wires (3a ∼ 3h) are substantively good to be circular, i.e. round, in a cross-sectional view.

On the other hand, each of the second wires (5a ∼ 5h) of the present embodiment has a recess in a contact portion (side surface) opposite to a corresponding one of the first wires adjacent to both sides of the second wire. For example, the second wire 5a has a recess 5ar in the contact portion (side surface) opposite to the first wire 3h, and a recess 5as in the contact portion (side surface) opposite to the first wire 3a as shown in FIG. 3.

The second wire 5b has a recess 5br in the contact portion (side surface) opposite to the first wire 3a, and a recess 5bs in the contact portion (side surface) opposite to the first wire 3b. Further, other second wires (5c, 5d, 5e, 5f, 5g, 5h) also have recesses 5cr, 5cs (not shown), ·····, 5hr (not shown), 5hs as will be easily understood.

The second wire (5a ∼ 5h) of the present embodiment has a convex Ogidai shape, i.e. annular segment shape or annular sector shape, (arch shape) in a cross-sectional view. For example, the second wire 5a has an Ogidai shape (arch shape) having a convex outer arc 5ap and a convex inner arc 5aq in the cross-sectional view shown in FIG. 3.

The second wire 5b has an Ogidai shape (arch shape) having a convex outer arc 5bp and a convex inner arc 5bq in the cross-sectional view. Further, the other second wires (5c, 5d, 5e, 5f, 5g, 5h) also have an Ogidai shape (arch shape) having a convex outer arc and a convex inner arc in the cross-sectional view was will be easily understood.

Incidentally, in the tubular body 1 of the present embodiment, the radially outer ends and radially inner ends of two second wires adjacent to both sides of a first wire are in contact with each other at a point of the outside and inside. For example, concerning the two second wires 5a and 5b adjacent to both sides of the first wire 3a, the two ends of the outer side are in contact with a point 7a, while the two ends of the inner side are also in contact with a point 9a as shown in FIGS. 1 to 3.

Thus, a helical pattern through point 7a is formed on the outer surface of the tubular body 1, and a helical pattern through point 9a is formed on the inner surface of the tubular body 1.

Similarly, as the radially outer ends and radially inner ends of any two second wires adjacent to each other at both sides of a corresponding one of the first wires (3b, 3c, 3d, 3e, 3f, 3g, 3h) are in contact with a point of the outer side and a point of the inner side, seven helical patterns by point 7b, 7c, 7d, 7e, 7f, 7g and 7h are formed on the outer surface of the tubular body 1, and seven helical patterns by point 9b, 9c, 9d, 9e, 9f, 9g and 9h are formed on the inner surface of the tubular body 1.

Materials of the first wires (3a ∼ 3h) and second wires (5a ∼ 5h) may be stainless steel, platinum alloys, Ni-Ti-based alloys, cobalt based alloys, or the like, but are not particularly limited to those materials. Any material having a biocompatibility may be used, although stainless steel is used in the present embodiment.

The first wires (3a ∼ 3h) and a second wires (5a ∼ 5h) may be formed of a same material as in the present embodiment, however they may also be formed of different materials. However, when the tubular body 1 is produced by a method using a swaging machine to be described later, it is preferred that a material of the second wires (5a ∼ 5h) is softer than a material of the first wires (3a ∼ 3h).

According to the tubular body 1 of the present embodiment, as the tubular body 1 is configured by twisting eight first wires (3a ∼ 3h) and eight second wires (5a ∼ 5h) in a hollow shape, wherein each of the first wires (3a ∼ 3h) has a substantially circular, i.e. round, cross section, and each of the second wires (5a ∼ 5h) has a recess (5ar, 5as, 5br, 5bs, 5cr ··· 5hs) in the contact portion (side surface) opposite to a corresponding one of the first wires adjacent to it, and has an Ogidai shape, i.e. annular segment shape or annular sector shape, (arch shape) having a convex outer arc (5ap, 5bp, ···, 5hp (not shown)) and a convex inner arc (5aq, 5bq, ··· 5hq (not shown)) in a cross-sectional view, it is possible to prevent a deviation between the first wires and second wires and improve a torque transmissibility and pushing force to the other end portion of the tubular body 1 when the user manipulates one end portion of the tubular body 1. Further, since the inner surface of the tubular body 1 is flat in a cross-sectional view, insertability of other medical devices into an inside of the tubular body 1 may be improved.

In the present embodiment, the number of the first wires and second wires is eight, but is not limited to eight. Two or more first wires and two or more second wires may be used in the tubular body. That is, the tubular body may comprise a plurality of first wires and a plurality of second wires.

### (Second Embodiment)

Next, a second embodiment of the present invention will be described.

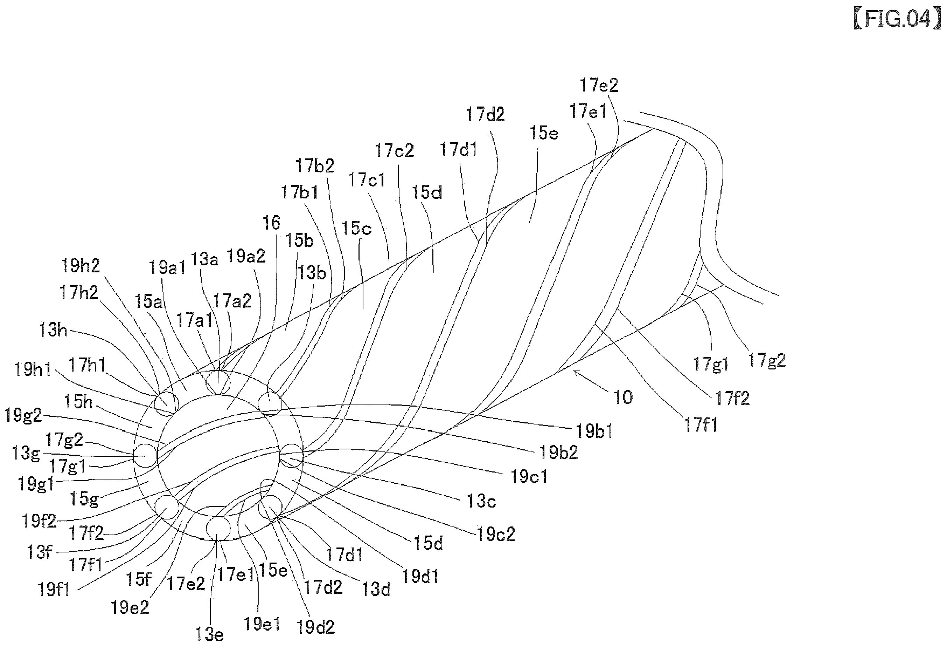

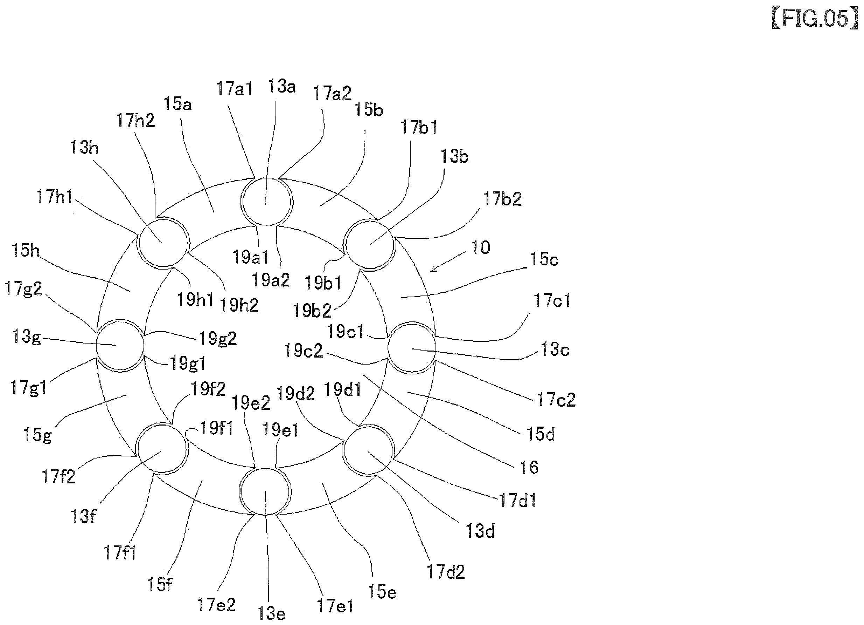

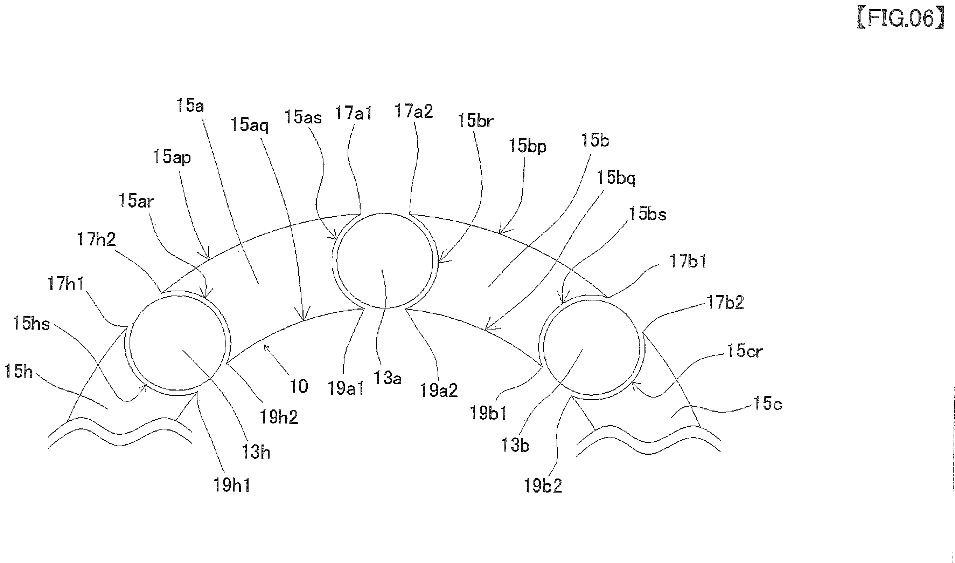

Figure 4 is a perspective view of the tubular body of the second embodiment, FIG. 5 is a cross-sectional view of the tubular body of the second embodiment, FIG. 6 is a partially enlarged view of FIG.5.

As shown in FIG. 4, a tubular body 10 is an elongated tubular body configured from eight first wires (13a, 13b, 13c, 13d, 13e, 13f, 13g, 13h) and eight second wires (15a, 15b, 15c, 15d, 15e, 15f, 15g, 15h) which are spirally twisted into a hollow tubular configuration so that the first wires and second wires are alternately arranged with each other and each of the first wires is placed between two adjacent second wires.

Further, the tubular body 10 includes a substantially circular outer peripheral surface and a substantially circular inner peripheral surface in a cross-sectional view, and the tubular body 10 forms a hollow portion 16 in an inside of the inner peripheral surface thereof.

Each of the first wires (13a ∼ 13h) of the present embodiment is substantially circular, i.e. round, in a cross-sectional view. Here, the reason why the first wires (13a ∼ 13h) are described as substantially circular and not exactly circular in a cross-sectional view depends on considering a case where the first wire is slightly deformed by the manufacturing method of the tubular body described below. However, the first wires (13a ∼ 13h) are substantively good to be circular, i.e. round, in a cross-sectional view.

On the other hand, each of the second wires (15a ∼ 15h) of the present embodiment has a recess in a contact portion (side surface) opposite to a corresponding one of the first wires adjacent to both sides of the second wire. For example, the second wire 15a has a recess 15ar in the contact portion (side surface) opposite to the first wire 13h, and a recess 15as in the contact portion (side surface) opposite to the first wire 13a as shown in FIG. 6.

The second wire 15b has a recess 15br in the contact portion (side surface) opposite to the first wire 13a, and a recess 15bs in the contact portion (side surface) opposite to the first wire 13b. Further, other second wires (15c, 15d, 15e, 15f, 15g, 15h) also have recesses 15cr, 15cs (not shown), ·····, 15hr (not shown), recess 15hs as will be easily understood.

The second wire (15a ∼ 15h) of the present embodiment has a convex Ogidai shape, i.e. annular segment shape or annular sector shape, (arch shape) in a cross-sectional view. For example, the second wire 15a has an Ogidai shape (arch shape) having a convex outer arc 15ap and a convex inner arc 15aq in the cross-sectional view as shown in FIG. 6.

The second wire 15b has an Ogidai shape (arch shape) having a convex outer arc 15bp and a convex inner arc 15bq in the cross-sectional view. Further, the other second wires (15c, 15d, 15e, 15f, 15g, 15h) also have an Ogidai shape (arch shape) having a convex outer arc and a convex inner arc in the cross-sectional view as will be easily understood.

Incidentally, the tubular body 10 of the present embodiment differs from the tubular body 1 of the first embodiment in that the radially outer ends and radially inner ends of two second wires adjacent to both sides of a first wire are separated from each other. For example, an outer end 17a1 of the second wire 15a adjacent to one side of the first wire 13a, and an outer end 17a2 of the second wire 15b adjacent to the other side of the first wire 13a are spaced apart from each other, further, an inner end 19a1 of the second wire 15a adjacent to one side of the first wire 13a, and an inner end 19a2 of the second wire 15b adjacent to the other side of the first wire 13a are also spaced apart from each other as shown in FIGS. 4 to 6.

Similarly, in the other first wires 13b ∼ 13h, an outer end 17b1 and an outer end 17b2, an outer end 17c1 and an outer end 17c2, ···, an outer end 17g1 and an outer end 17g2, an outer end 17h1 and an outer end 17h2 are also spaced apart from each other, and an inner end 19b1 and an inner end 19b2, an inner end 19c1 and an inner end 19c2, ···, an inner end 19g1 and an inner end 19g2, an inner end 19h1 and the inner end 19h2 are also spaced apart.

Accordingly, two helical patterns per one first wire are formed on the outer surface and the inner surface of the tubular body 10 as shown in FIG. 4. In the present embodiment, a total of 16 helical patterns are formed on the outer surface of the tubular body10, and a total of 16 helical patterns are formed on the inner surface of the tubular body 10.

Similar to the first wires (3a ∼ 3h) and second wires (5a ∼ 5h) of the first embodiment, materials of the first wires (13a ∼ 13h) and second wires (15a ∼ 15h) may be stainless steel, platinum alloys, Ni-Ti-based alloys, cobalt based alloys, or the like, but are not particularly limited to those materials. Any material having biocompatibility may be used, although stainless steel is used in the present embodiment.

The first wires (13a ∼ 13h) and second wires (15a ∼ 15h) may be formed of a same material as in the present embodiment, however they may also be formed of different materials. However, when tubular body 10 is produced by a method using a swaging machine to be described later, it is preferred that a material of the second wires (15a ∼ 15h) is softer than a material of the first wires (13a ∼ 13h).

Here, a method for manufacturing the tubular body 10 will be described.

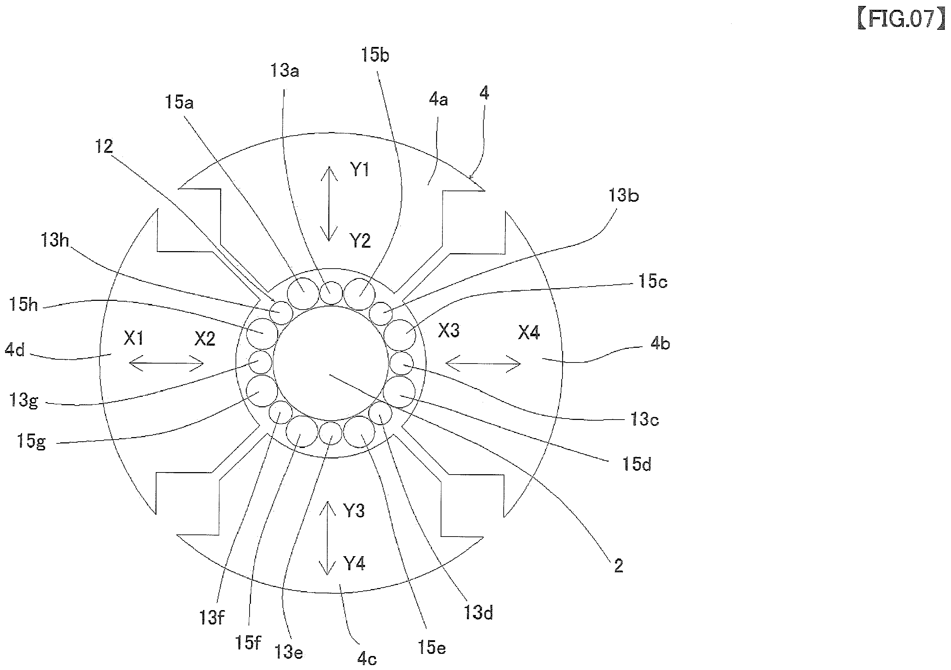

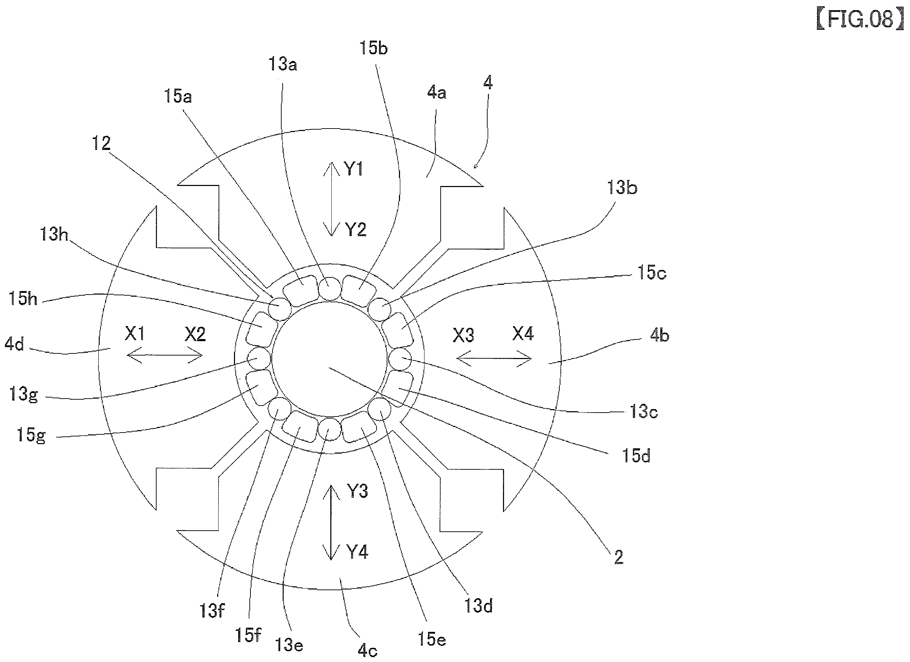

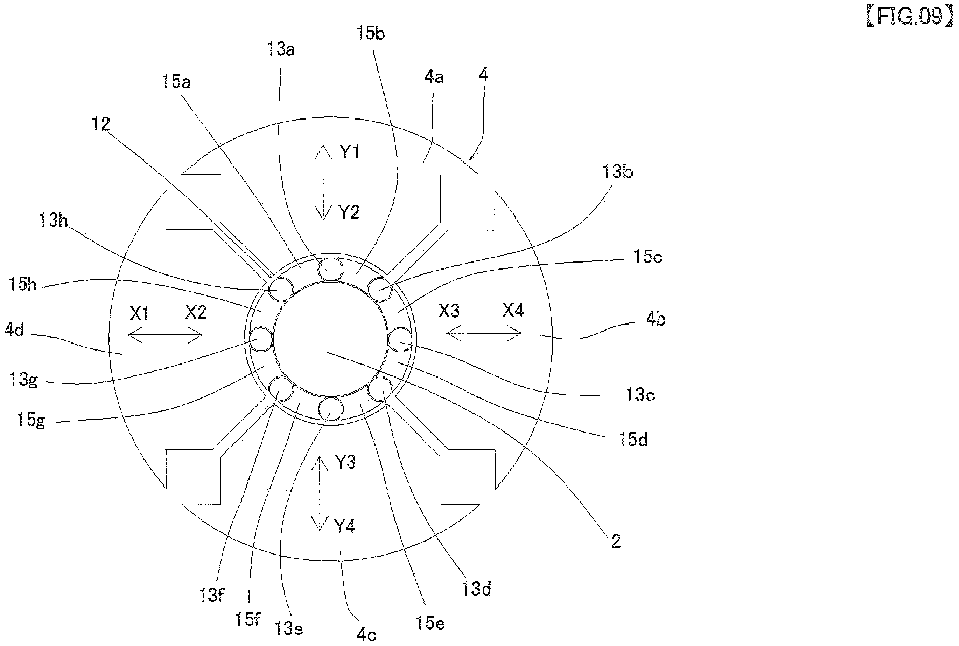

FIG. 7 is a cross-sectional view showing an initial state of setting a stranded wire to a swaging machine in producing the tubular body of the second embodiment, FIG. 8 is a cross-sectional view showing an intermediate state in producing the tubular body of the second embodiment, and FIG. 9 is a cross-sectional view showing a final state in producing the tubular body of the second embodiment.

Incidentally, a swaging machine used to produce the tubular body 10 may be any swaging machine among two-split die, three-split die, four-splits die and six-slits die. In the present embodiment, a manufacturing method will be described using the swaging machine with a four-split die.

To produce the tubular body 10, firstly, a manufacturer prepares a stranded wire 12 configured by winding eight first wires (13a ∼ 13h) of circular cross-section and eight second wires (15a ∼ 15h) of circular cross-section, wherein a diameter of each second wire is larger than a diameter of each first wire, in a spiral manner around a circumference of a core wire 2.

Then, as shown in FIG. 7, the manufacturer sets the stranded wire 12 to a swaging machine 4 and deforms the stranded wire 12 by operating the swaging machine 4. Specifically, the manufacturer deforms the stranded wire 12 by vibrating a first die 4a in a direction toward Y1 and Y2, a second die 4b in a direction toward X3 and X4, a third die 4c in direction toward Y3 and Y4, and a fourth die 4d in a direction toward X1 and X2 respectively while rotating a first die 4a, a second die 4b, a third die 4c and a fourth die 4d of swaging machine 4 around the stranded wire 12.

When the manufacturer continues to operate the swaging machine 4 for a predetermined time in this way, a cross-sectional shape of each of eight first wires (13a ∼ 13h) remains circular, while a cross-sectional shape of each of eight second wires (15a ∼ 15h) is deformed into an Ogidai shape (arch shape) having a larger diameter than each of the first wires (13a ∼ 13h) as shown in FIG. 8.

Further when the manufacturer continues to operate the swaging machine 4 for a predetermined time in this way, a cross-sectional shape of each of eight first wires (13a ∼ 13h) remains circular, while a cross-sectional shape of each of the eight second wires (15a ∼ 15h) having a larger diameter than each of the first wires(13a ∼ 13h) is deformed into a convex Ogidai shape (arch shape) in a cross-sectional view, having recesses in the contact portions adjacent to a first wire adjacent to both sides as shown in FIG. 9.

Then, the tubular body 10 is completed by pulling out the core wire 2 from the stranded wire 12, after removing the stranded wire 12 from the swaging machine 4.

On the other hand, without removing the stranded wire 12 from the swaging machine 4, when the manufacturer continues to operate the swaging machine 4 for a further predetermined time, a stranded wire 12 is completed where the radially outer ends and radially inner ends of two second wires adjacent to both sides of a first wire (13a ∼ 13h) contact each other at a point on the outside and inside of the stranded wire in a cross-sectional view. Then, the tubular body 1 as shown in FIGS. 1 to 3 is completed by pulling out the core wire 2 from the stranded wire 12, after removing the stranded wire 12 from the swaging machine 4.

According to the tubular body 10 of the present embodiment, as the tubular body 10 is configured by twisting eight first wires (13a ∼ 13h) and eight second wires (15a ∼ 15h) in a hollow shape, wherein each first wire (13a ∼ 13h) has a substantially circular cross section, and each second wire (15a ∼ 15h) has a recess (15ar, 15as, 15br, 15bs, 15cr ··· 15hs) in a contact portion (side surface) opposite to a corresponding one of the first wires adjacent to it, and has an Ogidai shape (arch shape) having convex outer arcs (15ap, 15bp, ···, 15hp (not shown)) and inner arcs (15aq, 15bq, ··· 15hq (not shown)) in a cross-sectional view, it is possible to prevent a deviation between the first wires and the second wires and improve a torque transmissibility and pushing force to the other end portion of the tubular body 10 when the user manipulates one end portion of the tubular body 10.

Further, since the inner surface of the tubular body 10 is flat in a cross-sectional view, insertability of other medical devices into an inside of the tubular body 10 may be improved.

Furthermore, the tubular body 10 of the present embodiment differs from the tubular body 1 of the first embodiment in that the radially outer ends and radially inner of two second wires adjacent to both sides of a first wire are separated from each other, so that the tubular body 10 may have a somewhat flexible structure compared to the tubular body 1 of the first embodiment.

In the present embodiment, the number of the first wires and second wires is eight, but is not limited to eight. Two or more first wires and two or more second wires may be used in the tubular body. That is, the tubular body may comprise a plurality of first wires and a plurality of second wires.

### (Third Embodiment)

Next, a description of a third embodiment of the present invention will be described.

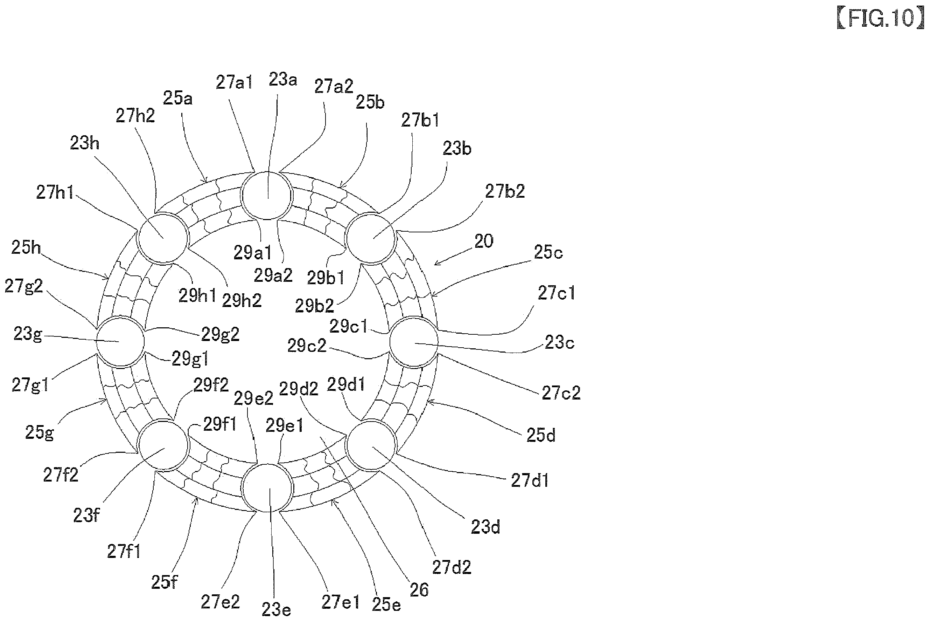

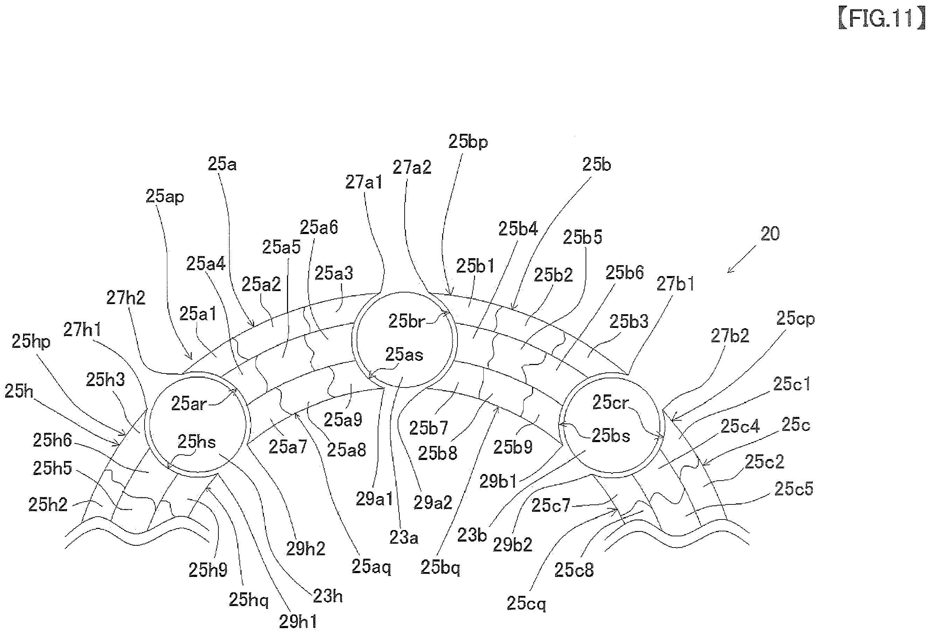

Figure 10 is a cross-sectional view of the tubular body of the third embodiment, FIG. 11 is a partially enlarged view of FIG. 10.

As shown in FIG. 10, a tubular body 20 is an elongated tubular body configured from eight first wires (23a, 23b, 23c, 23d, 23e, 23f, 23g, 23h) and eight second wires (25a, 25b, 25c, 25d, 25e, 25f, 25g, 25h) which are spirally twisted into a hollow configuration so that the first wires and second wires are alternately arranged with each other and each of the first wires is placed between two adjacent second wires.

The tubular body 20 of the present embodiment differs from the tubular body 10 of the second embodiment in that each of the eight second wires (25a ∼ 25h) is a multi-stranded wire configured from nine third wires twisted with each other. Specifically, the second wire 25a is configured from nine third wires (25a1, 25a2, 25a3, 25a4, 25a5, 25a6, 25a7, 25a8, 25a9), and the second wire 25b is configured from nine third wires (2Sb1, 25b2, 25b3, 25b4, 25b5, 25b6, 25b7, 25b8, 25b9) as shown in FIG. 11.

Similarly, each of the other second wires 25c ∼ 25h is configured from nine third wires (2Sc1 ∼ 25c9, 25d1 ∼ 25d9, 25e1 ∼ 25e9, 25f1 ∼ 25f9, 25g1 ∼ 25g9, 25h1 ∼ 25h9 twisted with each other (not one part shown)).

Further, the tubular body 20 includes a substantially circular outer peripheral surface and a substantially circular inner peripheral surface in a cross-sectional view, and the tubular body 20 forms a hollow portion 26 in an inside of the inner peripheral surface thereof.

Each of the first wires (23a ∼ 23h) of the present embodiment is substantially circular, i.e. round, in a cross-sectional view. Here, the reason why the first wires (23a ∼ 23h) are described as substantially circular and not exactly circular in a cross-sectional view depends on considering a case where the first wire is slightly deformed by the manufacturing method of the tubular body described above. However, the first wires (23a ∼ 23h) are substantively good to be circular, i.e. round, in a cross-sectional view.

On the other hand, each of the second wires (25a ∼ 25h) of the present embodiment has a recess in a contact portion (side surface) opposite to a corresponding one of the first wires adjacent to both sides of the second wire. For example, the second wire 25a has a recess 25ar in the contact portion (side surface) opposite to the first wire 23h, and a recess 25as in the contact portion (side surface) opposite to the first wire 23a as shown in FIG. 11.

The second wire 25b has a recess 25br in the contact portion (side surface) opposite to the first wire 23a, and a recess 25bs in the contact portion (side surface) opposite to the first wire 23b. Further, each of the other second wires (25c, 25d, 25e, 25f, 25g, 25h) also has recesses 25cr, 25cs (not shown), ·····, 25hr (not shown), 25hs as will be easily understood.

Each second wire (25a ∼ 25h) of the present embodiment has a convex Ogidai shape, i.e. annular segment shape or annular sector shape, (arch shape) in a cross-sectional view. For example, the second wire 25a has an Ogidai shape (arch shape) having a convex outer arc 25ap and a convex inner arc 25aq in the cross-sectional view shown in FIG. 11.

The second wire 25b has an Ogidai shape (arch shape) having a convex outer arc 25bp and a convex inner arc 25bq in the cross-sectional view. Further, the other second wires (25c, 25d, 25e, 25f, 25g, 25h) also have an Ogidai shape (arch shape) having a convex outer arc and a convex inner arc in the cross-sectional view as will be easily understood.

Incidentally, the tubular body 20 of the present embodiment is similar to the tubular body 10 of the second embodiment in that the radially outer ends and radially inner ends of two second wires adjacent to both sides of a first wire are separated from each other. For example, an outer end 27a1 of the second wire 25a adjacent to one side of the first wire 23a, and an outer end 27a2 of the second wire 25b adjacent to the other side of the first wire 23a are spaced apart from each other, further, an inner end 29a1 of the second wire 25a adjacent to one side of the first wire 23a, and an inner end 29a2 of the second wire 25b adjacent to the other side of the first wire 23a are also spaced apart from each other as shown in FIGS. 10 and 11.

Similarly, in the other first wire 23b ∼ 23h, an outer end 27b1 and an outer end 27b2, an outer end 27c1 and an outer end 27c2, ···, an outer end 27g1 and an outer end 27g2, an outer end 27h1 and an outer end 27h2 are spaced apart from each other, an inner end 29b1 and an inner end 29b2, an inner end 29c1 and an inner end 29c2, ···, an inner end 29g1 and an inner end 29g2, an inner end 29h1 and the inner end 29h2 are also spaced apart from each other.

Accordingly, two helical patterns per one first wire are formed on the outer surface and inner surface of the tubular body 20 as shown in FIG. 4. In the present embodiment, a total of 16 helical patterns are formed on the outer surface of the tubular body 20, and a total of 16 helical patterns are formed on the inner surface of the tubular body 20.

Similar to the first wires and second wires of the first embodiment and second embodiment, the materials of the first wires (23a ∼ 23h) and second wires (25a ∼ 25h) may be stainless steel, platinum alloys, Ni-Ti-based alloys, cobalt based alloys, and the like, but are not particularly limited to those materials. Any material having a biocompatibility may be used, although stainless steel is used in the present embodiment.

The first wires (23a ∼ 23h) and second wires (25a ∼ 25h) may be formed of a same material as in the present embodiment, however they may also be formed of different materials. However, when the tubular body 20 is produced by the method using a swaging machine as described above, it is preferred that a material of the second wires (25a ∼ 25h) is softer than a material of the first wires (23a ∼ 23h).

Note that the manufacturing method of the tubular body 20 is identical to the manufacturing method of the tubular body 10 of the second embodiment. However, instead of the second wires (15a ∼ 15h) in FIG. 7, a stranded wire configured by twisting nine third wires each having a circular cross-section is used.

That is, the tubular body 20 of this embodiment is produced by preparing a stranded wire configured by winding eight first wires (23a ∼ 23h) of circular cross-section and eight second wires (25a ∼ 25h) of substantially circular cross-section, wherein a diameter of a second wire is larger than a diameter of a first wire (23a ∼ 23h), in a spiral manner around the circumference of a core wire 2.

Then, when the manufacturer continues to operate the swaging machine 4 for a predetermined time, a cross-sectional shape of each of eight first wires (23a ∼ 23h) remains circular, while a cross-sectional shape of each of eight second wires (25a ∼ 25h) having a larger diameter than each of the first wires (23a ∼ 23h) has recesses in the contact portions adjacent to a first wire adjacent to both sides and is deformed into an Ogidai shape (arch shape) as shown in FIG. 10. Thereafter, similarly to the manufacturing method of the tubular body 10 of the second embodiment, by extracting the core wire 2 from the stranded wire, the tubular body 20 is completed.

According to the tubular body 20 of the present embodiment, as the tubular body 20 is configured by twisting eight first wires (23a ∼ 23h) and eight second wires (25a ∼ 25h) in a hollow shape, wherein each of first wires (23a ∼ 23h) has a substantially circular cross section, each of second wires (25a ∼ 25h) has a recess (25ar, 25as, 25br, 25bs, 25cr ··· 25hs) in a contact portion (side surface) opposite to a corresponding one of the first wires adjacent to it, and has an Ogidai shape (arch shape) having convex outer arcs (25ap, 25bp, ···, 25hp (not shown)) and inner arcs (25aq, 25bq, ··· 25hq (not shown)) in a cross-sectional view, and further each of second wires (25a ∼ 25h) is configured by twisting nine third wires (such as 25a1 ∼ 25a9) with each other, it is possible to prevent a deviation between the first wires and the second wires and improve a torque transmissibility and pushing force to the other end portion of the tubular body 20 when the user manipulates one end portion of the tubular body 20.

Further, since the inner surface of the tubular body 20 is flat in a cross-sectional view, insertability of other medical devices into an inside of the tubular body 20 may be improved. And when the user bends the tubular body 20, a subtle movement of the third wires may improve flexibility and durability of the tubular body.

In the present embodiment, the number of the first wires and second wires is eight, but is not limited to eight. Two or more first wires and two or more second wires may be used in the tubular body. That is, the tubular body may comprise a plurality of first wires and a plurality of second wires.

In the present embodiment, although the second wire is configured by twisting nine third wires, it is not limited to nine. Three or more third wires may be used in the tubular body.

### (Fourth Embodiment)

Next, a description of a fourth embodiment of the present invention will be described.

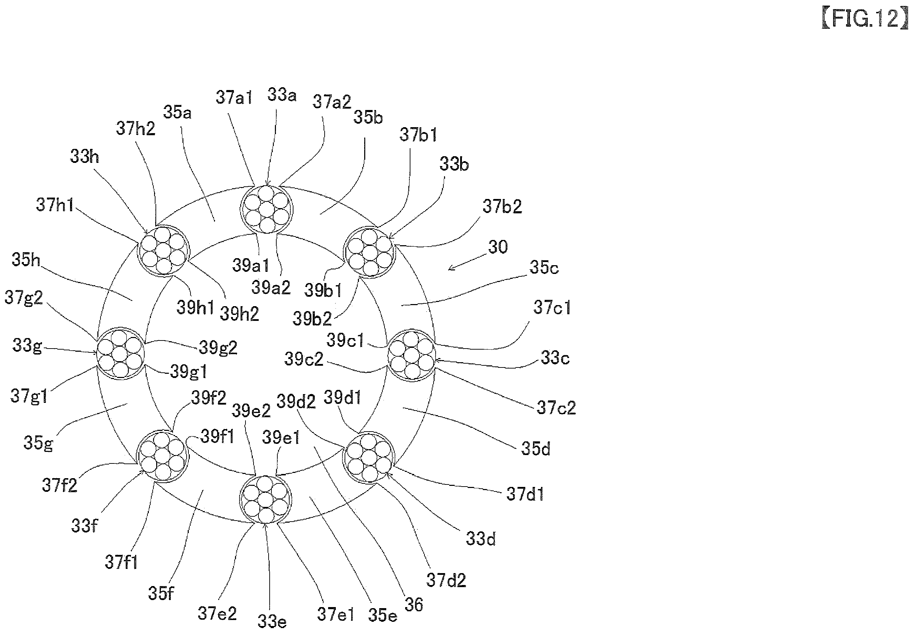

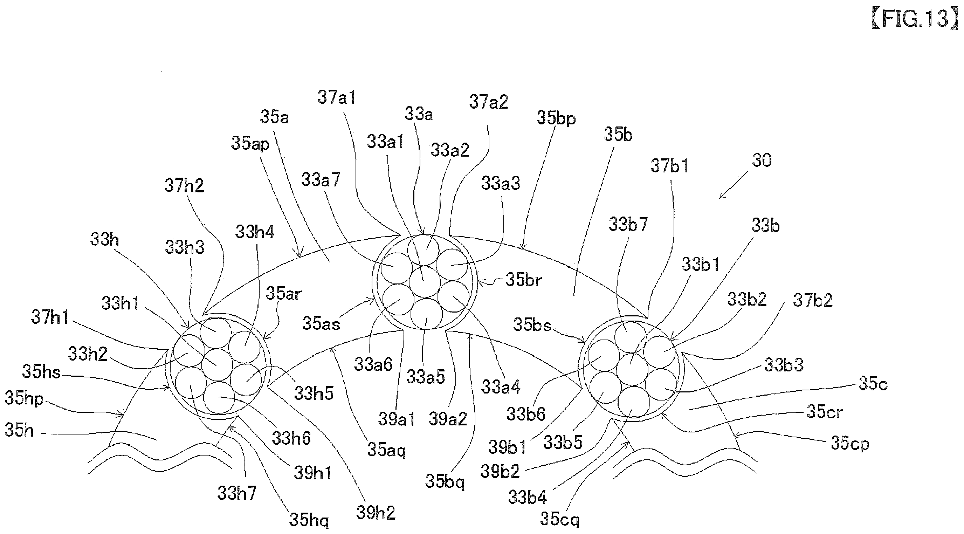

Figure 12 is a cross-sectional view of the tubular body of the fourth embodiment, FIG. 13 is a partially enlarged view of FIG. 12.

As shown in FIG. 12, a tubular body 30 is an elongated tubular body configured from eight first wires (33a, 33b, 33c, 33d, 33e, 33f, 33g, 33h) and eight second wires (35a, 35b, 35c, 35d, 35e, 35f, 35g, 35h) which are spirally twisted into a hollow tubular configuration so that the first wires and second wires are alternately arranged with each other and each of the first wires is placed between two adjacent second wires.

The tubular body 30 of the present embodiment differs from the tubular body 10 of the second embodiment in that each of the first wires (33a ∼ 33h) is configured from seven fourth wires twisted with each other. Specifically, the first wire 33a is configured from seven fourth wires (33a1, 33a2, 33a3, 33a4, 33a5, 33a6, 33a7), the first wire 3b is configured from seven fourth wires (33b1, 33b2, 33b3, 33b4, 333b5, 33b6, 33b7) as shown in FIG. 13.

Similarly, the other first wires 33c ∼ 33h are each configured from seven fourth wires (33c1 ∼ 33c7, 33d1 ∼ 33d7, 33e1 ∼ 33e7, 33f1 ∼ 33f7, 33g1 ∼ 33g7, 33h1 ∼ 33h7 (not one part shown)).

Further, the tubular body 30 includes a substantially circular outer peripheral surface and a substantially circular inner peripheral surface in a cross-sectional view, and the tubular body 30 forms a hollow portion 36 in an inside of the inner peripheral surface thereof.

Further, each of the second wires (35a ∼ 35h) of the present embodiment has a recess in a contact portion (side surface) opposite to a corresponding one of the first wires adjacent to both sides of the second wire. For example, the second wire 35a has a recess 35ar in a contact portion (side surface) opposite to the first wire 33h, and a recess 35as in a contact portion (side surface) opposite to the first wire 33a as shown in FIG. 13.

The second wire 35b has a recess 35br in a contact portion (side surface) adjacent to the first wire 33a, and a recess 35bs in a contact portion (side surface) opposite to the first wire 33b. Further, the other second wires (35c, 35d, 35e, 35f, 35g, 35h) also have recesses 35cr, 35cs (not shown), ·····, 35hr (not shown), 35hs as will be easily understood.

A second wire (35a ∼ 35h) of the present embodiment has a convex Ogidai shape, i.e. annular segment shape or annular sector shape, (arch shape) in a cross-sectional view. For example, the second wire 35a has an Ogidai shape (arch shape) having a convex outer arc 35ap and a convex inner arc 35aq in the cross-sectional view shown in FIG. 13.

The second wire 35b has an Ogidai shape (arch shape) having a convex outer arc 35bp and a convex inner arc 35bq in the cross-sectional view. Further, the other second wires (35c, 35d, 35e, 35f, 35g, 35h) also have an Ogidai shape (arch shape) having a convex outer arc and a convex inner arc in a cross-sectional view as will be easily understood.

Incidentally, the tubular body 30 of the present embodiment is similar to the tubular body 10 of the second embodiment in that the radially inner ends and radially outer ends of two second wires adjacent to both sides of a first wire are separated from each other. For example, an outer end 37a1 of the second wire 35a adjacent to one side of the first wire 33a, and an outer end 37a2 of the second wire 35b adjacent to the other side of the first wire 33a are spaced apart from each other, further, an inner end 39a1 of the second wire 35a adjacent to one side of the first wire 33a, and an inner end 39a2 of the second wire 35b adjacent to the other side of the first wire 33a are also spaced apart from each as shown in FIGS. 12 and 13.

Similarly, in the other first wires 33b ∼ 33h, an outer end 37b1 and an outer end 37b2, an outer end 37cl and an outer end 37c2, ···, an outer end 37g1 and an outer end 37g2, an outer end 37h1 and an outer end 37h2 are also spaced apart from each other, and an inner end 39bl and an inner end 39b2, an inner end 39c1 and an inner end 39c2, ···, an inner end 39g1 and an inner end 39g2, an inner end 39h1 and the inner end 39h2 are also spaced apart from each other.

Accordingly, two helical patterns per one first wire are formed on the outer surface and inner surface of the tubular body 30 as shown in FIG. 4. In the present embodiment, a total of 16 helical patterns are formed on the outer surface of the tubular body 30, and a total of 16 helical patterns are formed on the inner surface of the tubular body 30.

Similar to the first wires and second wires of the first embodiment to third embodiment, the materials of the first wires (33a ∼ 33h) and second wires (35a ∼ 35h) may be stainless steel, platinum alloys, Ni-Ti-based alloys, cobalt based alloys, or the like, but are not particularly limited to those materials. Any material having a biocompatibility may be used, although stainless steel is used in the present embodiment.

The first wires (33a ∼ 33h) and second wires (35a ∼ 35h) may be formed of a same material as in the present embodiment, but they may also be formed of different materials. However, when the tubular body 30 is produced by a method using the swaging machine described before, it is preferred that a material of the second wires (35a ∼ 35h) is softer than a material of the first wires (33a ∼ 33h).

Note that the manufacturing method of the tubular body 30 is identical to the manufacturing method of the tubular body 10 of the second embodiment. However, instead of the first wire (13a ∼ 13h) shown in FIG. 7, a stranded wire configured by twisting seven fourth wires each having a circular cross-section is used.

That is, the tubular body 30 of this embodiment is produced by preparing a stranded wire configured by winding eight first stranded wires (33a ∼ 33h) which are each configured from seven fourth wires of circular cross-section, and eight second wires (35a ∼ 35h) of circular cross-section, wherein a diameter of a second wire is larger than a diameter of a first wire (33a ∼ 33h), in a spiral manner around the circumference of a core wire 2.

Then, when the manufacturer continues to operate the swaging machine 4 for a predetermined time, a cross-sectional shape of each of eight first wires (33a ∼ 33h) remains circular, while a cross-sectional shape of each of eight second wires (35a ∼ 35h) having a larger diameter than each of the first wires (33a ∼ 33h) has recesses in the contact portions adjacent to a first wire adjacent to both sides, and is deformed into an Ogidai shape (arch shape) as shown in FIG. 12. Thereafter, similarly to the manufacturing method of the tubular body 10 of the second embodiment, by extracting the core wire 2 from the stranded wire the tubular body 30 is completed.

According to the tubular body 30 of the present embodiment, as the tubular body 30 is configured by twisting eight first wires (33a ∼ 33h) and eight second wires (35a ∼ 35h) into a hollow shape, wherein each of first wires (33a ∼ 33h) has a substantially circular cross section, each of second wires (35a ∼ 35h) has a recess (35ar, 35as, 35br, 35bs, 35cr ··· 35hs) in a contact portion (side surface) opposite to a corresponding one of the first wires adjacent to it, and has an Ogidai shape (arch shape) having convex outer arcs (35ap, 35bp, ···, 35hp (not shown)) and inner arcs (35aq, 35bq, ··· 35hq (not shown)) in a cross-sectional view, and further each of the first wires (33a ∼ 33h) is configured by twisting seven fourth wires (such as 33a1 ∼ 33a7), it is possible to prevent a deviation between the first wires and the second wires and improve a torque transmissibility and pushing force to the other end portion of the tubular body 30 when the user manipulates one end portion of the tubular body 30.

Further, since the inner surface of the tubular body 30 is flat in a cross-sectional view, insertability of other medical devices into an inside of the tubular body 30 may be improved. And when the user bends the tubular body 30, a subtle movement of the fourth wires may improve flexibility and durability of the tubular body.

In the present embodiment, the number of the first wires and second wires is eight, but is not limited to eight. Two or more first wires and two or more second wires may be used in the tubular body. That is, the tubular body may comprise a plurality of first wires and a plurality of second wires.

In the present embodiment, although a first wire is configured by twisting seven fourth wires, it is not limited to seven. Three or more fourth wires may be used in the tubular body.

### (Fifth Embodiment)

Next, a description of a fifth embodiment of the present invention will be described.

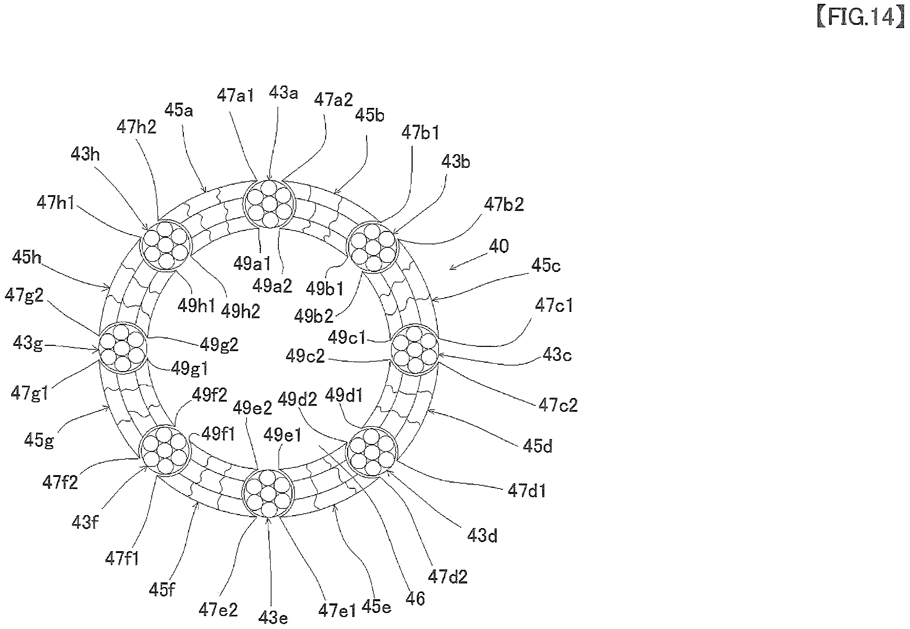

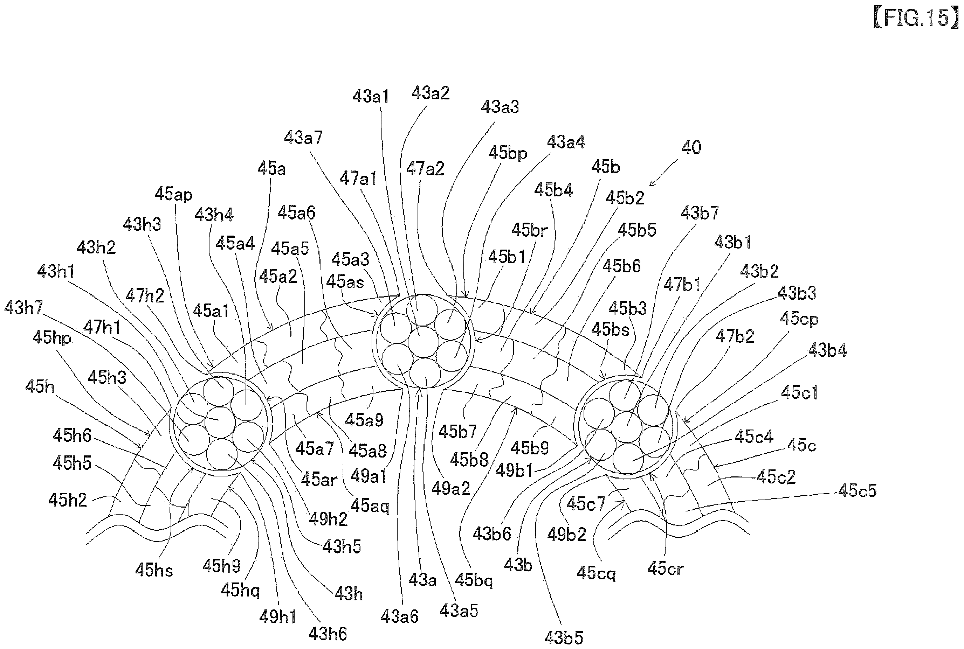

Figure 14 is a cross-sectional view of the tubular body of the fifth embodiment, FIG. 15 is a partially enlarged view of FIG. 14.

As shown in FIG. 14, a tubular body 40 is an elongated tubular body configured from eight first wires (43a, 43b, 43c, 43d, 43e, 43f, 43g, 43h) and eight second wires (45a, 45b, 45c, 45d, 45e, 45f, 45g, 45h) which are spirally twisted into a hollow tubular configuration so that the first wires and second wires are alternately arranged with each other and each of the second wires is placed between two adjacent first wires.

The tubular body 40 of the present embodiment differs from the tubular body 10 of the second embodiment in that each of the first wires (43a ∼ 43h) is configured from seven fourth wires twisted with each other, and each of the second wires (45a ∼ 45h) is configured from twisting nine third wires with each other.

Specifically, the first wire 43a is configured from seven fourth wires (43a1, 43a2, 43a3, 43a4, 43a5, 43a6, 43a7), and the second wire 45a is configured from nine third wires (45a1, 45a2, 45a3, 45a4, 45a5, 45a6, 45a7,45a8,45a9) as shown in FIG. 15.

Similarly, the other first wires 43b ∼ 43h are each configured from seven fourth wires (43b1 ∼ 43b7,43c1 ∼ 43c7, 43d1 ∼ 43d7, 43e1 ∼ 43e7, 43f1 ∼ 43f7, 43g1 ∼ 43g7, 43h1 ∼ 43h7 (not one part shown)), while the other second wires 45b ∼ 45h are each configured from nine third wires (45b1∼ 45b9,45c1 ∼ 45c9, 45d1 ∼ 45d9, 45e1 ∼ 45e9, 45f1 ∼ 45f9, 45g1 ∼ 45g9, 45h1 ∼ 45h9 (not one part shown)).

Further, the tubular body 40 includes a substantially circular outer peripheral surface and a substantially circular inner peripheral surface in a cross-sectional view, and the tubular body 40 forms a hollow portion 46 in an inside of the inner peripheral surface thereof.

Further, each of the second wires (45a ∼ 45h) of the present embodiment has a recess in a contact portion (side surface) opposite to a corresponding one of the first wires adjacent to both sides of the second wire. For example, the second wire 45a has a recess 45ar in a contact portion (side surface) opposite to the first wire 43h, and a recess 45as in a contact portion (side surface) opposite to the first wire 43a as shown in FIG. 15.

The second wire 45b has a recess 45br in a contact portion (side surface) opposite to the first wire 43a, and a recess 45bs in a contact portion (side surface) opposite to the first wire 43b. Further, the other second wires (45c, 45d, 45e, 45f, 45g, 45h) also have recesses 45cr, ·····, 45hs as will be easily understood.

Each second wire (45a ∼ 45h) of the present embodiment has a convex Ogidai shape, i.e. annular segment shape or annular sector shape, (arch shape) in a cross-sectional view. For example, the second wire 45a has an Ogidai shape (arch shape) having a convex outer arc 45ap and a convex inner arc 45aq in the cross-sectional view shown in FIG. 15.

The second wire 45b has an Ogidai shape (arch shape) having a convex outer arc 45bp and a convex inner arc 45bq in the cross-sectional view. Further, the other second wires (45c, 45d, 45e, 45f, 45g, 45h) also have an Ogidai shape (arch shape) having a convex outer arc and a convex inner arc in the cross-sectional view as will be easily understood.

Incidentally, the tubular body 40 of the present embodiment is similar to the tubular body 10 of the second embodiment in that the radially outer ends and radially inner ends of two second wires adjacent to both sides of a first wire are separated from each other. For example, an outer end 47a1 of the second wire 45a adjacent to one side of the first wire 43a, and an outer end 47a2 of the second wire 45b adjacent to the other side of the first wire 43a are spaced apart from each other, further, an inner end 49a1 of the second wire 45a adjacent to one side of the first wire 43a, and an inner end 49a2 of the second wire 45b adjacent to the other side of the first wire 43a are also spaced apart from each other as shown in FIGS. 14 and 15.

Similarly, in the other first wires 43b ∼ 43h, an outer end 47b1 and an outer end 47b2, an outer end 47c1 and an outer end 47c2, ···, an outer end 47g1 and an outer end 47g2, an outer end 47h1 and an outer end 47h2 are also spaced apart from each other, an inner end 49b1 and an inner end 49b2, an inner end 49c1 and an inner end 49c2, ···, an inner end 49g1 and an inner end 49g2, an inner end 49h1 and the inner end 49h2 are also spaced apart from each other.

Accordingly, two helical patterns per one first wire are formed on the outer surface and the inner surface of the tubular body 40 as shown in FIG. 4. In the present embodiment, a total of 16 helical patterns are formed on the outer surface of the tubular body 40, and a total of 16 helical patterns are formed on the inner surface of the tubular body 40.

Similar to the first wires and second wires of the first embodiment to fourth embodiment, the materials of the first wires (43a ∼ 43h) and second wire (45a ∼ 45h) may be stainless steel, platinum alloys, Ni-Ti-based alloys, cobalt based alloys, or the like, but are not particularly limited to those materials. Any material having a biocompatibility may be used, although stainless steel is used in the present embodiment.

The first wires (43a ∼ 43h) and second wires (45a ∼ 45h) may be formed of a same material as in the present embodiment, however they may also be formed of different materials. However, when the tubular body 40 is produced by a method using the swaging machine described before, it is preferred that a material of the second wires (45a ∼ 45h) is softer than a material of the first wires (43a ∼ 43h).

Note that the manufacturing method of the tubular body 40 is identical to the manufacturing method of the tubular body 10 of the second embodiment. However, instead of the first wires (13a ∼ 13h) in FIG. 7, a stranded wire configured by twisting seven fourth wires each having a circular cross-section is used. And instead of each of the second wires (15a ∼ 15h) in FIG. 7, a stranded wire configured by twisting nine third wires each having a circular cross-section is used.

That is, the tubular body 40 of this embodiment is produced by preparing a stranded wire configured by winding eight first stranded wires (43a ∼ 43h) which are each configured from seven fourth wires of circular cross-section, and eight second wires (45a ∼ 45h) of circular cross-section, wherein the diameter of a second wire is larger than a diameter of the first wire (43a ∼ 43h), in a spiral manner around the circumference of a core wire 2.

Then, when the manufacturer continues to operate the swaging machine 4 for a predetermined time, a cross-sectional shape of each of the eight first wires (43a ∼ 43h) remains circular, while a cross-sectional shape of each of the eight second wires (45a ∼ 45h) having larger diameter than the the first wires (43a ∼ 43h) has recesses in the contact portions adjacent to the first wires adjacent to both sides, is deformed into Ogidai shape (arch shape) as shown in FIG. 14. Thereafter, similarly to the manufacturing method of the tubular body 10 of the second embodiment, by extracting the core wire 2 from the stranded wire, the tubular body 40 is completed.

According to the tubular body 40 of the present embodiment, as the tubular body 40 is configured by twisting eight first wires (43a ∼ 43h) and eight second wires (45a ∼ 45h) in a hollow shape, wherein each of first wires (43a ∼ 43h) has a substantially circular cross section, each of second wires (45a ∼ 45h) has a recess (45ar, 45as, 45br, 45bs, 45cr ··· 45hs) in a contact portion (side surface) opposite to a corresponding one of the first wires adjacent to it, and has an Ogidai shape (arch shape) having convex outer arcs (45ap, 45bp, ···, 45hp (not shown)) and inner arcs (45aq, 45bq, ··· 45hq (not shown)) in the transverse plane radiation direction, and further each of first wires (43a ∼ 43h) is configured by twisting seven fourth wires (such as 43a1 ∼ 43a7), it is possible to prevent a deviation between the first wires and second wires and improve a torque transmissibility and pushing force to the other end portion of the tubular body 40 when the user manipulates one end portion of the tubular body 40.

Further, since the inner surface of the tubular body 40 is flat in a cross-sectional view, insertability of other medical devices into an inside of the tubular body 40 may be improved. And when the user bends the tubular body 40, a subtle movement of the third wires and a subtle movement of the fourth wires may improve flexibility and durability of the tubular body.

In the present embodiment, the number of the first wires and second wires is eight each, but is not limited to eight. Two or more first wires and two or more second wires may be used in the tubular body. That is, the tubular body may comprise a plurality of first wires and a plurality of second wires.

In the present embodiment, although a first wire is configured by twisting seven fourth wires, it is not limited to seven. Three or more fourth wires may be used in the tubular body.

In the present embodiment, although a second wire is configured by twisting nine third wires, it is not limited to nine. Three or more third wires may be used in the tubular body.

### [Description of the code]

1,10, 20, 30, 40 ··· tubular body
2 ··· core wire
3a ∼ 3h, 13a ∼ 13h, 23a ∼ 23h, 33a ∼ 33h, 43a ∼ 43h ··· first wires
4 ··· swaging machine
5a ∼ 5h, 15a ∼ 15h, 25a ∼ 25h, 35a ∼ 35h, 45a ∼ 45h ··· second wires
6,16,26,36,46 ··· hollow portion
7a ∼ 7h, 17a ∼ 17h, 27a ∼ 27h, 37a ∼ 37h, 47a ∼ 47h ··· outer ends
9a ∼ 9h, 19a ∼ 19h, 29a ∼ 29h, 39a ∼ 39h, 49a ∼ 49h ··· inner ends

## Claims

1. A tubular body (1) comprising:
a plurality of first wires (3a, 3b, 3c, 3d, 3e, 3f, 3g, 3h), and
a plurality of second wires (5a, 5b, 5c, 5d, 5e, 5f, 5g, 5h), wherein
the first wires (3a, 3b, 3c, 3d, 3e, 3f, 3g, 3h) and the second wires (5a) are twisted into a tubular configuration so that the first wires (3a, 3b, 3c, 3d, 3e, 3f, 3g, 3h) and the second wires (5a, 5b, 5c, 5d, 5e, 5f, 5g, 5h) are alternately arranged with each other and each of the second wires (5a, 5b, 5c, 5d, 5e, 5f, 5g, 5h) is placed between two adjacent first wires (3a, 3b, 3c, 3d, 3e, 3f, 3g, 3h), and wherein
at least one of the first wires (3a) has a round cross section perpendicular to a longitudinal direction of the tubular body, **characterized in that**
at least one of the second wires (5a) has a cross section perpendicular to the longitudinal direction with a recess (5as) in a side surface opposite to the first wire (3a), and the cross section of the second wire (5a) is annular sector shaped and projects radially outwardly.

2. The tubular body (20) according to claim 1, wherein
each of the first wires (3a, 3b, 3c, 3d, 3e, 3f, 3g, 3h) has a round cross section perpendicular to the longitudinal direction, and
each of the second wires (5a) has a cross section perpendicular to the longitudinal direction with a recess (5as) in one side surface opposite to the first wire (3a) and another recess (5ar) in the other side surface opposite to the other first wire (3h), and the cross section of each of the second wires (5a) is annular sector shaped and projects radially outwardly.

3. The tubular body (20) according to claim 1 or 2, wherein
each of the second wires (25a, 25b, 25c, 25d, 25e, 25f, 25g, 25h) is a multi-strand wire configured from a plurality of third wires (25a1, 25a2, 25a3, 25a4, 25a5, 25a6, 25a7, 25a8, 25a9) twisted with each other.

4. The tubular body (30) according to any one of claims 1 to 3, wherein
each of the first wires (33a, 33b, 33c, 33d, 33e, 33f, 33g, 33h) is a multi-strand wire configured from a plurality of fourth wires (33a1, 33a2, 33a3, 33a4, 33a5, 33a6, 33a7, 33a8, 33a7) twisted with each other.

5. The tubular body (1) according to any one of claims 1 to 4, wherein
each of the second wires (5a, 15a, 25a, 35a, 45a) is made of a material that is softer than a material of the first wires (3a, 13a, 23a, 33a, 43a).

6. The tubular body (1) according to claim 2, wherein
a radially outer end of the recess (5as) of the second wire (5a) contacts with a radially outer end of the recess (5br) of the adjacent second wire (5b), and
a radially inner end of the recess (5as) of the second wire (5a) contacts with a radially inner end of the recess (5br) of the adjacent second wire (5b).

7. The tubular body (10) according to claim 2, wherein
a radially outer end (17a1) of the recess (15as) of the second wire (15a) is spaced apart from a radially outer end (17a2) of the recess (15br) of the adjacent second wire (15b), and
a radially inner end (19a1) of the recess (15as) of the second wire (15a) is spaced apart from a radially inner end (19a2) of the recess (15br) of the adjacent second wire (15b).

8. A method for manufacturing a tubular body (10) according to any one of claims 1 to 7, the method comprising:
twisting a plurality of first wires (13a, 13b, 13c, 13d, 13e, 13f, 13g, 13h) having a round cross section perpendicular to a longitudinal direction of the tubular body, and a plurality of second wires (15a, 15b, 15c, 15d, 15e, 15f, 15g, 15h) having a round cross section perpendicular to the longitudinal direction around a core wire (2) so that the first wires (13a, 13b, 13c, 13d, 13e, 13f, 13g, 13h) and the second wires (15a, 15b, 15c, 15d, 15e, 15f, 15g, 15h) are alternately arranged with each other and each of the second wires (15a, 15b, 15c, 15d, 15e, 15f, 15g, 15h) is placed between two of the first wires (13a, 13b, 13c, 13d, 13e, 13f, 13g, 13h), wherein a diameter of the round cross section of the second wires (15a, 15b, 15c, 15d, 15e, 15f, 15g, 15h) is greater than a diameter of the round cross section of the first wires (13a, 13b, 13c, 13d, 13e, 13f, 13g, 13h);
pressing the second wires (15a) from radially outside so that the round cross section of each of the second wires (15a) is deformed into an annular sector shape projecting radially outwardly and having a recess (15as) in a side surface opposite to the first wire (13a); and
pulling the core wire (2) from the tubular body (10).
